# EUROPEAN PATENT APPLICATION

(11) **EP 4 618 090 A1**
(43) Date of publication of application: **17.09.2025**
(21) Application number: 23892093.8
(22) Date of filing: 20.11.2023
(51) Int. Cl.: G16C 20/70, G06N 20/00

(54) **DEVICE AND METHOD FOR TRAINING DATA GENERATION THROUGH OBJECT DIVERSIFICATION OF STRUCTURAL FORMULA**

(30) Priority: 18.11.2022 KR 20220155035
(71) Applicant: LG Management Development Institute Co., Ltd., Seoul 07336 (KR)
(72) Inventor: JO, Ah Ra, Seoul 07796 (KR); JO, Yeon Sik, Seoul 07796 (KR); PARK, Chang Young, Seoul 07796 (KR); LEE, Soon Young, Seoul 07796 (KR)
(74) Representative: BCKIP Part mbB
(86) International application number: PCT/KR2023/018694
(87) International publication number: WO 2024/107031

(57) **Abstract**

A training data generation device according to an embodiment of the present invention comprises a processor and at least one memory electrically connected to the processor. The at least one memory stores: one or more feature variables determining the features of objects expressing structural formulas; and a first setpoint set determined in advance for each of the one or more feature variables. The processor: loads first chemical formula data, including information about at least one piece of node and information about at least one piece of edge, and the one or more feature variables; sets setpoints of the one or more feature variables as the first setpoint set; generates a first structural formula on the basis of the first chemical formula by using an object set as the first setpoint set that is applied; acquires, in response to an input changing the setpoints of one or more feature variables, a second setpoint set in which the setpoints of the corresponding feature variables are changed; generates a second structural formula by using an object set as the second setpoint set that is applied; and generates the training data including the generated second structural formula.

## Description

### [TECHNICAL FIELD]

The present invention relates to a device and a method for training data generation through object diversification of a structural formula. More particularly, the present invention relates to a device and a method for training data generation, which are capable of mass-producing a structural formula by diversifying setpoints of objects constituting the structural formula based on chemical formula data including node and edge information.

### [BACKGROUND ART]

In developing an artificial intelligence model that recognizes a structural formula, generating training data using only an existing structural formula generation program does not reflect the diversity of actual structural formula data, and thus it is difficult to expect high performance of the artificial intelligence model.

In the conventional structural formula generation program, the structural formula can be generated only in a fixed format, and it is difficult to diversify a thickness, a color, or an interval of line segments representing a bond of the structural formula, or a font, a size, a color, or the like of characters representing atoms.

In addition, in order to improve the performance of the artificial intelligence model, a large amount of training data is required, and since the conventional generation program can generate only one image at a time, it takes a lot of time to process data to generate additional data.

In addition, the conventional generation program suffers from the difficulty that in training an artificial intelligence model using a vision technology, coordinate information and class information of atoms and bonding regions need to be derived from the structural formula training data, but such information cannot be acquired by the conventional chemical structural formula representation generation program. Acquiring coordinate information and class information of an atom and a bonding region by hand is difficult.

### [DETAILED DESCRIPTION OF INVENTION]

### [TECHNICAL PROBLEMS]

The present invention provides a device and a method for generating training data, which may mass-produce various formats of training data in order to train an artificial intelligence model which recognizes a structural formula.

The objects to be achieved by the present invention are not limited to the aforementioned objects, and other objects, which are not mentioned above, will be apparent to a person having ordinary skill in the art from the following description.

### [TECHNICAL SOLUTION]

According to an aspect of the present invention, a device for training data generation may include: a processor and at least one memory electrically connected to the processor, and the at least one memory may store at least one feature variable for determining a feature of an object representing a structural formula, and a first setpoint set predetermined for each of the at least one feature variable, and the processor may load first chemical formula data including information about at least one node and information about at least one edge, and the at least one feature variable, set a setpoint of the at least one feature variable to the first setpoint set, generate, based on the first chemical formula data, a first structural formula by using an object set that is applied to the first setpoint set, acquire, according to an input of changing the setpoint of the at least one feature variable, a second setpoint set in which the setpoint of the corresponding feature variable is changed, generate a second structural formula using an object set that is applied to the second setpoint set, and generate training data including the generated second structural formula.

In addition, according to another aspect of the present invention, a method for training data generation, which is performed by a training data generation device may include: storing, by the training data generation device, at least one feature variable for determining a feature of an object representing a structural formula, and a first setpoint set predetermined for each of the at least one feature variable, and loading first chemical formula data including information about at least one node and information about at least one edge, and the at least one feature variable; setting a setpoint of the at least one feature variable to the first setpoint set; generating, based on the first chemical formula data, a first structural formula by using an object set that is applied to the first setpoint set; acquiring, according to an input of changing the setpoint of the at least one feature variable, a second setpoint set in which the setpoint of the corresponding feature variable is changed; generating a second structural formula using an object set that is applied to the second setpoint set; and generating training data including the generated second structural formula.

In addition, a computer program stored in a computer-readable recording medium, coupled with a device that is hardware, to execute the method for generating training data of claim 9 may be further provided.

### [EFFECT OF INVENTION]

According to the present invention, various types of structural formula training data that are not fixed formats can be generated. Further, a large amount of structural formula training data can be generated from one structural formula data.

In addition, a setpoint may be assigned to the structural formula generated by the training data generation device of the present invention for each feature variable of an object constituting the structural formula. This facilitates annotation with a set value assigned to each object, can reduce an error occurrence rate of an annotation task, and can also reduce a cost in a step of verifying whether an annotation task result is correctly performed.

Therefore, it is possible to improve the performance of the artificial intelligence model that recognizes the structural formula by utilizing the structural formula generated by the training data generation device of the present invention.

The effects of the present invention are not limited to the aforementioned effect, and other effects, which are not mentioned above, will be apparent to a person having ordinary skill in the art from the following disclosure.

### [BRIEF DESCRIPTION OF THE DRAWING]

FIG. 1 is a block diagram of a device for training data generation according to an embodiment of the present invention.
FIG. 2 is a diagram for describing a process of generating a structural formula based on chemical formula data according to an embodiment of the present invention.
FIG. 3A illustrates a first structural formula generated by a predetermined setpoint, and FIG. 3B illustrates a second structural formula in which setpoints of some characters, numbers, and symbols are changed according to an embodiment of the present invention.
FIG. 4A illustrates a first structural formula generated by a predetermined setpoint, and FIG. 4B illustrates a second structural formula in which setpoints of some figures are changed according to an embodiment of the present invention.
FIG. 5A illustrates a first structural formula generated by a predetermined setpoint, and FIG. 5B illustrates a second structural formula in which setpoints of some figures are changed according to an embodiment of the present invention.
FIG. 6A illustrates a first structural formula generated by a predetermined setpoint, and FIG. 6B illustrates a second structural formula in which setpoints of some figures are changed according to an embodiment of the present invention.
FIG. 7A illustrates a first structural formula generated by a predetermined setpoint, and FIG. 7B illustrates a second structural formula in which setpoints of some figures are changed according to an embodiment of the present invention.
FIG. 8A illustrates a first structural formula generated by a predetermined setpoint, and FIG. 8B illustrates a second structural formula in which setpoints of some figures are changed according to an embodiment of the present invention.
FIG. 9A illustrates a first structural formula generated by a predetermined setpoint, and FIGS. 9B and 9C illustrate a second structural formula in which setpoints of some figures are changed according to an embodiment of the present invention.
FIG. 10 illustrates a structural formula in which objects constituting a node are annotated according to an embodiment of the present invention.
FIG. 11 illustrates a structural formula in which objects constituting an edge are annotated according to an embodiment of the present invention.
FIG. 12 illustrates a type of an object constituting the edge according to an embodiment of the present invention.
FIG. 13 illustrates a structural formula in which objects constituting a bracket are annotated according to an embodiment of the present invention.
FIG. 14 illustrates a structural formula in which a point where the bracket and the object intersect is annotated according to an embodiment of the present invention.
FIG. 15 is a block diagram for describing a method for generating training data according to an embodiment of the present invention.

### [BEST MODE FOR CARRYING OUT THE INVENTION]

Advantages and features of the present invention, and methods for accomplishing the same will be more clearly understood from embodiments described in detail below with reference to the accompanying drawings. However, the present invention is not limited to the embodiments set forth below, and may be embodied in various different forms. The present embodiments are just for rendering the disclosure of the present invention complete and are set forth to provide a complete understanding of the scope of the invention to a person with ordinary skill in the technical field to which the present invention pertains, and the present invention will only be defined by the scope of the claims.

It is also to be understood that the terms used in this specification are for the purpose of describing embodiments only and is not intended to limit the present invention. In this specification, the singular form also includes the plural form, unless the context indicates otherwise. It is to be understood that the terms "comprise" and/or "comprising" used in the specification do not exclude the presence or addition of one or more other components other than stated components. Like reference numerals refer to like components throughout the specification and "and/or" includes respective mentioned components and all one or more combinations of the components. Although the terms "first", "second", and the like are used for describing various components, these components are not confined by these terms. These terms are merely used for distinguishing one component from another component. Therefore, a first component to be mentioned below may be a second component in a technical concept of the present invention.

Unless otherwise defined, all terms (including technical and scientific terms) used in the present specification may be used as the meaning which may be commonly understood by the person with ordinary skill in the art, to which the present invention pertains. Further, terms defined in commonly used dictionaries should not be interpreted in an idealized or excessive sense unless expressly and specifically defined.

Terms used in this specification are described.

An object represents a group of elements for representing a structural formula. For example, the structural formula may consist of characters, numbers, symbols, and figures. Specifically, the characters may represent atoms or compounds constituting the structural formula, the numbers may represent the number of atoms or charges, the symbols represent positive charges (+), negative charges (-), or brackets representing specific repeating compound groups, and the figures may represent single line segments, double line segments, triple line segments, dotted lines, and the like representing chemical bonds.

The structural formula in this specification may be understood as one graph. The graph is a data structure consisting of points and lines connecting the points. Here, the point is referred to as a node, and the line is referred to as an edge. In other words, the structural formula may be understood as one graph in which at least one atom or compound corresponding to the node is linked by at least one bond corresponding to the edge. The structural formula may also include the bracket. The brackets represent a group of atoms and bonds repeated in the structural formula.

Hereinafter, embodiments of the present invention will be described with reference to drawings.

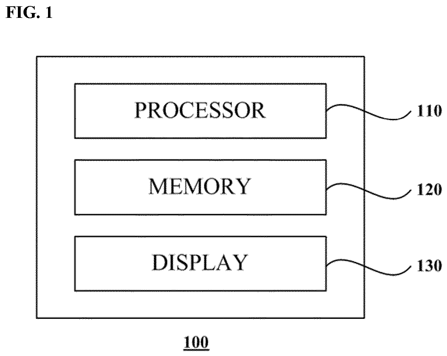

FIG. 1 is a block diagram of a device for training data generation according to an embodiment of the present invention.

The training data generation device 100 may include a processor 110, a memory 120, and a display 130. The processor 110, the memory 120, and the display 130 may be electrically connected to each other.

The processor 110 may load a first chemical formula data 200 including at least one node information 210 and at least one edge information 220 from the memory 120 or an external device. The processor 110 may also load the at least one feature variable 300 from the memory 120 or the external device.

The processor 110 may set, for the at least one loaded feature variable 300, a setpoint of each feature variable 300 to a predetermined first setpoint set 400.

The processor 110 may generate a structural formula in a form in which atoms or compounds are chemically bonded, based on at least one node information and at least one edge information included in structure data. Therefore, the processor 110 may generate, based on first chemical formula data 200, a first structural formula by using an object set and applied to the predetermined first setpoint set 400.

The processor 110 acquires, according to an input for changing the setpoint of the at least one feature variable 300, a second setpoint set 500 or a second setpoint set 600 in which the setpoint of a corresponding feature variable is changed.

An input of changing the setpoint of the feature variable 300 may be input from a user. In addition, the setpoint of the feature variable 300 may be randomly changed by the processor 110. In this case, the setpoint of each feature variable 300 may be randomly changed within a predetermined range or condition. The processor 110 may connect any structural formula to the first structural formula.

In this way, a large amount of structural formula training data having various shapes may be generated.

Further, the user's input and a processing of the processor 110 may be combined to change the setpoint of the feature variable 300. For example, when the user selects one specific variable among the feature variables 300, the processor 110 may randomly change the setpoint of the feature variable within a predetermined range. In addition, when the user inputs the number of structural formulas to be generated, the processor 110 may randomly change any feature variable so that the corresponding number of structural formulas is generated. In this way, a large amount of structural formula training data having shapes desired by the user may be generated.

The processor 110 may acquire the second setpoint set 500 or the second setpoint set 600 in which a setpoint of at least one of the feature variables 300 is changed.

The processor 110 may generate a second structural formula using an object set and applied to the second setpoint set 500 or the second setpoint set 600. The processor 110 may generate training data including the generated second structural formula.

The memory 120 may store at least one feature variable 300 for determining a feature of an object representing a structural formula, and a first setpoint set 400 predetermined for each of the at least one feature variable 300.

The display 130 may output the structural formula. Specifically, the display 130 may output the first structural formula generated by using the object set and applied to the first setpoint set 400, and the display 130 may output the second structural formula generated by using the object set and applied to a changed second setpoint set 500 or second setpoint set 600.

In addition, the display 130 may output the first chemical formula data 200, the feature variable 300, the predetermined first setpoint set 400, and the changed second setpoint sets 500 and 600. The user may change the feature variable 300 of the desired object after confirming the first structural formula through the display 130, and confirm the changed second structural formula.

In addition, the display 130 may output the second structural formula separately for each time point at which the changed second setpoint set is acquired.

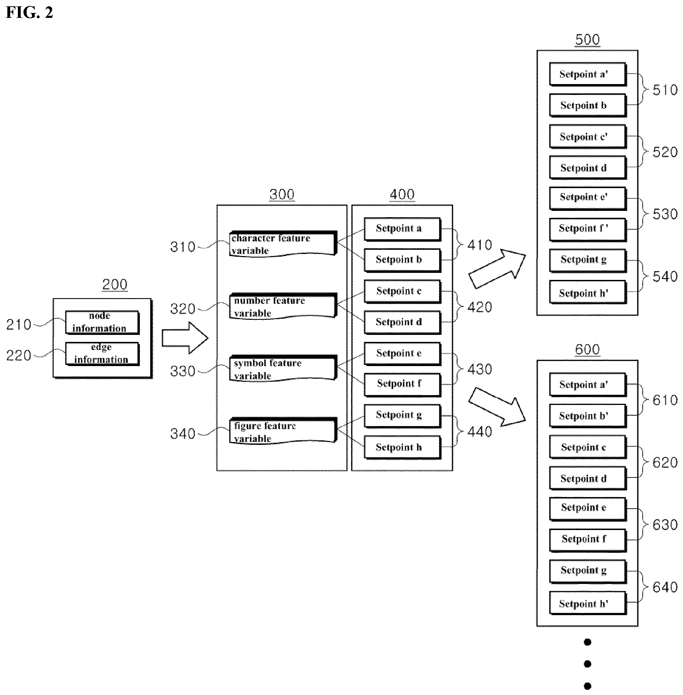

FIG. 2 is a diagram for describing a process of generating a structural formula based on chemical formula data according to an embodiment of the present invention.

FIG. 2 illustrates a process of generating various types of structural formulas based on the loaded first chemical formula data 200. Specifically, a process of setting a value of a specific variable 300 of an object for representing the first chemical formula data 200 as a structural formula to a predetermined first setpoint set 400, acquiring the second setpoint set 500 or the second setpoint set 600 according to an input of changing the setpoint of the specific variable 300, and generating the structural formula using the object set to the second setpoint set 500 or the second setpoint set 600 is illustrated.

The first chemical formula data 200 includes at least one node information 210 and at least one edge information 220. For example, the first chemical formula data 200 may include a list of atoms constituted by the structural formula, a multiplicity (single, double, and triple) of atoms linked by chemical bonds and the chemical bonds, coordinates on a 2D or 3D space for each atom, the number of atoms, the number of chemical bonds, the total charge of the structural formula, a charge of each atom and attributes related to isomers, and the like. The first chemical formula data 200 may be a format of an MOL file.

The feature variable 300 may include a character feature variable 310, a number feature variable 320, a symbol feature variable 330, and a figure feature variable 340.

The character feature variable 310 may include a position of a character, a font type, an outline thickness of the character, a size of each character, a size by word, a spacing between characters, a color of the character, and an angle at which the character is tilted.

The number feature variable 320 may include a position of a number, a font type, an outline thickness of the number, a size of the number, a spacing between the number and the character, a spacing between numbers, a color of the number, and an angle at which the number is tilted.

The symbol feature variable 330 may include a position of a symbol, a font type, an outline thickness of the symbol, a size of the symbol, a spacing between the symbol and the character, a spacing between the symbol and the number, a spacing between symbols, a color of the symbol, and an angle at which the symbol is tilted.

The figure feature variable 340 may include a position of a figure, a type, an outline thickness of the figure, a size of the figure, a spacing between the figure and the character, a spacing between the figure and the number, a spacing between the figure and the symbol, a spacing between figures, a color of the figure, and an angle at which the figure is tilted.

By providing various feature variables 300 in this way, it is possible to generate a large amount of structural formulas from one chemical formula data. Meanwhile, it is understood that, in addition to the variables listed above, all variables that determine the feature of the object constituting the structural formula may be included in the feature variable 300.

The first setpoint set 400 is a group of predetermined setpoints. The first setpoint set 400 may include a setpoint 410 for the character feature variable 310, a setpoint 420 for the number feature variable 320, a setpoint 430 for the symbol feature variable 330, and a setpoint 440 for the figure feature variable 340. Although only eight setpoints of the setpoints a to h are illustrated in the drawings, it is understood that the setpoints are not limited thereto.

For example, the setpoint a may have a setting of "Arial" as a value for determining a font of the character, and the setpoint b may have a setting of "10" as a value of determining a size of the character.

The second setpoint set 500 is a set including setpoints in which the setpoints a, c, e, f, and h of the first setpoint set 400 are changed to setpoints a', c', e', f', and h', respectively. The second setpoint set 600 is a set including setpoints in which the setpoints a, b, and h of the first setpoint set 400 are changed to setpoints a', b', and h', respectively. The remaining setpoints which are not changed are the same as the setpoints of the first setpoint set 400.

One structural formula may be generated by using an object set and applied to the second setpoint set 500, and another type of structural formula may also be generated by using the object set and applied to the second setpoint set 600.

Although FIG. 2 illustrates a case where two second setpoint sets 500 and 600 are formed, it can be understood that numerous second setpoint sets may be formed according to a combination of changed setpoints.

Hereinafter, the present invention will be described using embodiments of specific structural formulas.

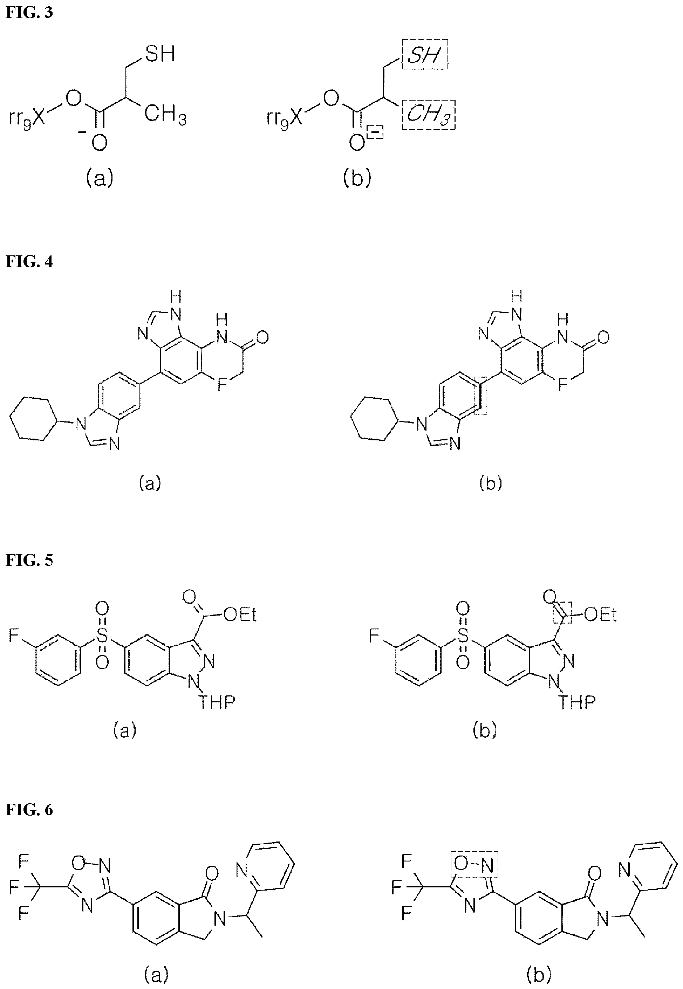

FIG. 3A illustrates a first structural formula generated by a predetermined setpoint, and FIG. 3B illustrates a second structural formula in which specific variable setpoints are changed for some characters, numbers, and symbols are changed according to an embodiment of the present invention. Specifically, a portion indicated by a dotted line in FIG. 3B shows an object changed in comparison with FIG. 3A.

As described above, the processor 110 may load the first chemical formula data 200 including node information and edge information, which may generate the structural formula of FIG. 3A, and the feature variable 300, and generate the first structural formula as illustrated in FIG. 3A by using the object set applied to the predetermined first setpoint set 400.

The processor 110 may acquire, according to an input for changing a setpoint of at least one feature variable 300, a second setpoint set 500 in which a setpoint of the feature variable is changed, and generate a second structure as illustrated in FIG. 3B by using an object set applied to the second setpoint set 500.

In FIG. 3A, SH represents a combination of characters, and CH3 represents a combination of a character and a number. Accordingly, the processor 110 may match the character feature variable 310 to the SH, and match the character feature variables 310 and the number feature variable 320 to the CH3, respectively. FIG. 3B illustrates a case where, for the SH, setpoints indicating the font and a tilting degree are changed within the character feature variable 310, and, for the CH3, setpoints indicating the font and the tilting degree within the character feature variables 310 and the number feature variables 320 are changed.

Further, a charge (-) of O in FIG. 3A is a symbol. Therefore, the charge (-) of O may be matched to the symbolic feature variable 330. FIG. 3B illustrated that a setpoint indicating a position of the charge (-) of O is changed.

As another embodiment, FIGS. 4 to 7 will be described.

FIG. 4A, FIG. 5A, FIG. 6A, and FIG. 7A illustrate the first structural formula generated by the predetermined first setpoint set 400, and FIG. 4B, FIG. 5B, FIG. 6B, and FIG. 7B illustrate the second structural formula in which setpoints of some figures are changed according to an embodiment of the present invention. Specifically, portions indicated by dotted lines in FIG. 4B, FIG. 5B, FIG. 6B, and FIG. 7B show objects changed in comparison with FIG. 4A, FIG. 5A, FIG. 6A, and FIG. 7A, respectively.

The combination of the first structural formulas illustrated in FIG. 4A, FIG. 5A, FIG. 6A, and FIG. 7A is a figure object. Therefore, the processor 110 may match the figure feature variable 340 to each combination.

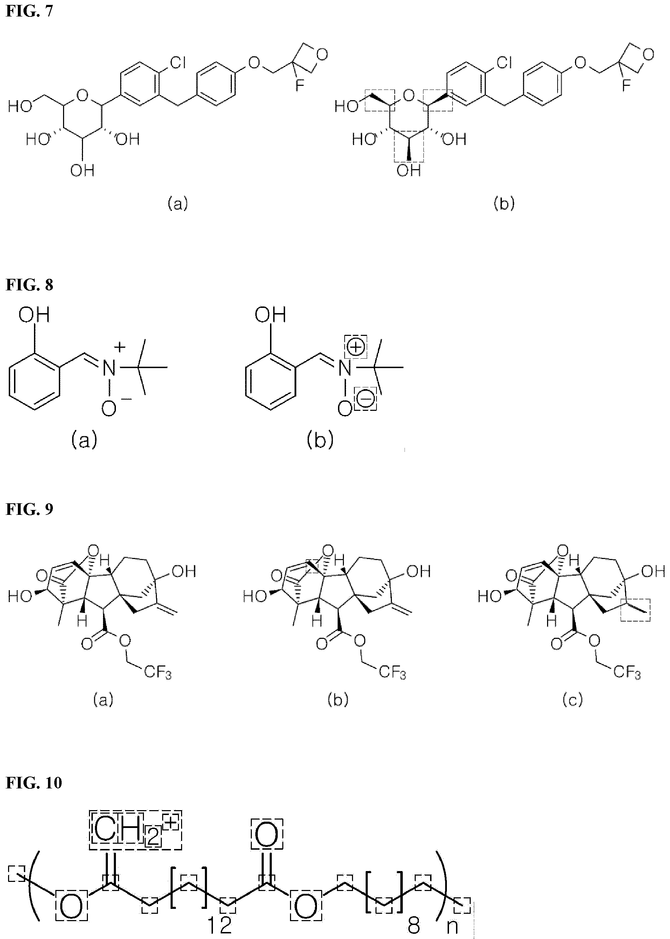

FIG. 4B illustrates a case where the setpoint indicating the thickness in the figure feature variable 340 is changed to increase with respect to a single combination of the dotted line indication portion. FIG. 5B illustrates a case where setpoint indicating a tilting angle of a figure and a spacing between the figures in the figure feature variable 340 are changed with respect to a double bond of the dotted line indication portion. FIG. 6B illustrates a case where a setpoint indicating a length in the figure feature variable 340 is changed to decrease with respect to a single bond of the dotted line indication portion. FIG. 7B illustrates a case where a setpoint indicating a type in the figure feature variable 340 is changed from a line segment to a triangle with respect to the single bond of the dotted line indication portion.

As another embodiment, FIG. 8 will be described.

FIG. 8A illustrates a first structural formula generated by a predetermined first setpoint set 400, and FIG. 8B illustrates a second structural formula in which setpoints of some figures are changed according to an embodiment of the present invention. Specifically, a portion indicated by a dotted line in FIG. 8B shows an object changed in comparison with FIG. 8A.

In FIG. 8A, charges (+ and -) of each atom represent a symbol object. Therefore, the processor 110 may match the symbol feature variable 330 to each symbol object. By changing a value of the matched symbol feature variable 330, a simple symbol (+, -) indicating charge as illustrated in FIG. 8A may be changed to a symbol illustrated in FIG. 8B. In addition, the positions of the symbols (+ and -) may also be changed as described above in the description of FIG. 3.

FIGS. 9A to 9C illustrate a case where two second structural formulas are generated from one first structural formula. FIG. 9B illustrates a case where the setpoint indicating the spacing between figures in the figure feature variable 340 is changed to increase with respect to an intersection between bonds in the dotted line indicated portion. FIG. 9C illustrates a case where a setpoint indicating a spacing between line segments in the figure feature variable 340 is changed to decrease with respect to a double bond of the dotted line indication portion.

As described above, various types of structural formulas may be generated in large quantities by changing predetermined setpoints for the feature variables 300 of characters, numbers, symbols, and figures that are objects constituting the structural formula.

In the second structure formula generated by the processor 110, a setpoint is set for each of feature variables 300 of objects constituting the second structural formula, and thus, the second structural formula may be utilized to annotate each object with the setpoint of the feature variable 300. Since each object is annotated, an artificial intelligence model that uses the annotated object as training data may improve a performance thereof.

In general, data annotation is performed by performing bounding box processing of an object included in an image, and inputting attribute information of the object subjected to the bounding box processing. Such an annotation is also referred to as data labeling. Then, a data set corresponding to a task result of the annotation is calculated in the form of a Java script object notation (JSON) file.

FIG. 10 illustrates a structural formula annotated to an object that constitutes a node of the second structural formula.

Dotted line portions shown in FIG. 10 means objects constituting the node. The feature variable 300 is matched to each of the objects, and a specific setpoint is set for each feature variable 300. For example, for O, C, and H, a setpoint of the character feature variable 310 may be set, for a number 2, a setpoint of the number feature variable 320 may be set, and for a node without a character and a number, a setpoint of the figure feature variable 340 may be set. As described above, the object constituting the node of the second structural formula may be formed by being annotated with a setpoint set for the feature variable 300 of the object.

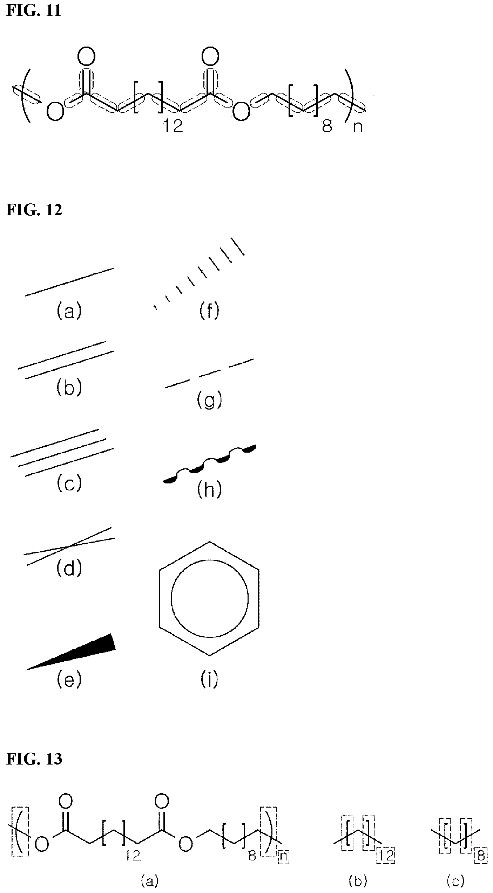

FIG. 11, FIG. 13A, and FIG. 14 illustrate the same second structural formula.

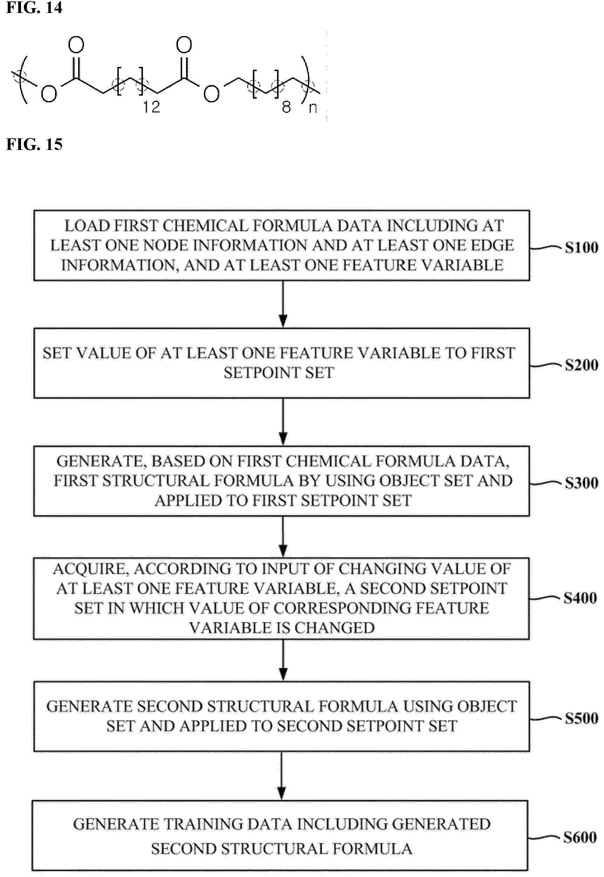

FIG. 11 illustrates a structural formula annotated to an object that constitutes an edge of the second structural formula. FIG. 13 illustrates a structural formula in which objects constituting a bracket of the second structural formula are annotated and FIG. 14 illustrates a structural formula in which a point where the object constituting the bracket of the second structural formula intersects with another object is annotated.

Dotted line portions shown in FIG. 11 mean objects constituting the edge. For example, for a single line segment and double line segments, a setpoint of the figure feature variable 340 may be set.

FIG. 12 illustrates a type of object constituting the edge according to an embodiment of the present invention. FIG. 12A illustrates a single bond, FIG. 12B illustrates a double bond, FIG. 12C illustrates a triple bond, FIG. 12D illustrates a form of a cross-linked bond, FIGS. 12E and 12F illustrate a form of a bond for three-dimensionally expressing a bond, FIG. 12G illustrates a bond indicated by a dotted line, FIG. 12H illustrates a wavy bond, and FIG. 12I illustrates a bond of a resonance structure.

As described above, the object constituting the edge of the second structural formula may be formed by being annotated with a setpoint set for the feature variable 300 of the object.

Dotted line portions shown in FIG. 13 mean objects constituting the bracket.

The bracket of FIG. 13A shows a structure in which an entire structural formula included in the corresponding bracket is linked while being repeated n times.

FIG. 13B illustrates a part of FIG. 13A. The bracket of FIG. 13B shows that the structural formula included in the bracket is linked while being repeated 12 times.

FIG. 13C illustrates a part of FIG. 13A. The bracket of FIG. 13C shows that the structural formula included in the bracket is linked while being repeated 8 times.

As described above, the object constituting the bracket of the second structural formula may be formed by being annotated with a setpoint set for the feature variable 300 of the object.

A dotted line portion shown in FIG. 14 indicates a point where the object constituting the bracket and the object constituting the edge intersect with each other. The intersection of the bracket and the edge may be annotated as illustrated in FIG. 14, separately from the intersection between the edge and the edge in FIG. 12D.

As described above, the object constituting the bracket and the object constituting the edge in the second structural formula may be formed by annotating a point where the object constituting the bracket and the object constituting an edge intersect with each other.

Such an annotation task may be automatically performed by the processor 110 of the training data generation device 100.

FIG. 15 is a block diagram for describing a method for generating training data according to an embodiment of the present invention.

The training data generation device 100 may load first chemical formula data 200 including at least one node information and at least one edge information, and at least one feature variable 300 (S100).

The training data generation device 100 may set a setpoint of the at least one feature variable 300 as a first setpoint set 400 (S200).

The training data generation device 100 may generate, based on the first chemical formula data 200, a first structural formula by using an object set and applied to the first setpoint set 400 (S300).

The training data generation device 100 acquires, according to an input of changing the setpoint of the at least one feature variable 300, a second setpoint set 500 in which the setpoint of the corresponding feature variable is changed (S400).

The training data generation device 100 may generate a second structural formula by using an object set and applied to the second setpoint set 500 (S500).

The training data generation device 100 may generate training data including the generated second structural formula (S600). In the second structural formula generated as described above, a setpoint set for each object may be annotated.

The memory may include at least one type of storage medium of a flash memory type storage medium, a hard disk type storage medium, a solid state disk (SSD) type storage medium, a silicon disk drive (SDD) type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk.

Meanwhile, the disclosed embodiments may be implemented in the form of a recording medium storing instructions executable by the computer. The instructions may be stored in the form of a program code and when the instructions are executed by a processor, the instructions generate a program module to perform operations of the disclosed embodiments. The recording medium may be implemented as a computer readable recording medium.

The computer readable recording medium includes all kinds of recording media storing instructions which may be deciphered by the computer. For example, the recording media may include a read only memory (ROM), a random access memory (RAM), a magnetic tape, a magnetic disk, a flash memory, an optical data storage device, etc.

As described above, the disclosed exemplary embodiments are described with reference to the accompanying drawings. Those skilled in the art to which the present invention pertains will understand that the present invention may be implemented in a different form the disclosed embodiments without changing the technical spirit or essential features of the present invention. The disclosed embodiments are exemplary and should not be construed as being limited.

### [Industrial Applicability]

The present invention relates to a training data generation device that machine-learns structural formula training data, and an artificial intelligence model trained by the training data generation device, and thus has an industrial applicability.

## Claims

1. A device for training data generation, the device comprising:
a processor; and
at least one memory electrically connected to the processor,
wherein the at least one memory stores at least one feature variable for determining a feature of an object representing a structural formula, and a first setpoint set predetermined for each of the at least one feature variable, and
wherein the processor
loads first chemical formula data including information about at least one node and information about at least one edge, and the at least one feature variable,
sets a setpoint of the at least one feature variable to the first setpoint set, generates, based on the first chemical formula data, a first structural formula by using an object set that is applied to the first setpoint set,
acquires, according to an input of changing the setpoint of the at least one feature variable, a second setpoint set in which the setpoint of the corresponding feature variable is changed,
generates a second structural formula using an object set that is applied to the second setpoint set, and
generates training data including the generated second structural formula.

2. The device of claim 1, wherein the object constituting a node of the second structural formula is formed by being annotated with the setpoint set for the feature variable of the corresponding object.

3. The device of claim 1, wherein the object constituting an edge of the second structural formula is formed by being annotated with the setpoint set for the feature variable of the corresponding object.

4. The device of claim 1, wherein an object constituting a bracket of the second structural formula is formed by being annotated with the setpoint set for the feature variable of the corresponding object.

5. The device of claim 1, wherein the object constituting the bracket and the object constituting the edge in the second structural formula are formed by annotating a point where the object constituting the bracket and the object constituting the edge intersect with each other.

6. The device of claim 1, wherein the training data generation device includes a display outputting a structural formula, and
wherein the display outputs the first structural formula and the second structural formula, and outputs the second structural formula separately for each time point at which the changed second setpoint set is acquired.

7. The device of claim 1, wherein the at least one feature variable includes at least one of a type, a thickness, a length, a size, and a color of the object, a spacing between objects, and an angle at which the object is tilted.

8. The device of claim 1, wherein the training data is used for training an artificial intelligence model for predicting chemical formula data including information about at least one node and information about at least one edge.

9. A method for training data generation, which is performed by a training data generation device, the method comprising:
storing, by the training data generation device, at least one feature variable for determining a feature of an object representing a structural formula, and a first setpoint set predetermined for each of the at least one feature variable, and
loading first chemical formula data including information about at least one node and information about at least one edge, and the at least one feature variable;
setting a setpoint of the at least one feature variable to the first setpoint set;
generating, based on the first chemical formula data, a first structural formula by using an object set and applied to the first setpoint set;
acquiring, according to an input of changing the setpoint of the at least one feature variable, a second setpoint set in which the setpoint of the corresponding feature variable is changed;
generating a second structural formula using an object set that is applied to the second setpoint set; and
generating training data including the generated second structural formula.

10. The method of claim 9, wherein an object constituting a node of the second structural formula is formed by being annotated with the setpoint set for the feature variable of the corresponding object.

11. The method of claim 9, wherein an object constituting an edge of the second structural formula is formed by being annotated with the setpoint set for the feature variable of the corresponding object.

12. The method of claim 9, wherein an object constituting a bracket of the second structural formula is formed by being annotated with the setpoint set for the feature variable of the corresponding object.

13. The method of claim 9, wherein an object constituting a bracket and an object constituting an edge in the second structural formula are formed by annotating a point where the object constituting the bracket and the object constituting the edge intersect with each other.

14. The method of claim 9, wherein the at least one feature variable includes at least one of a type, a thickness, a length, a size, and a color of the object, a spacing between objects, and an angle at which the object is tilted.

15. A computer program stored in a computer-readable recording medium, coupled with a device that is hardware, to execute the method for generating training data of claim 9.
